# EUROPEAN PATENT APPLICATION

(11) **EP 4 039 198 A1**
(43) Date of publication of application: **10.08.2022**
(21) Application number: 20886623.6
(22) Date of filing: 14.07.2020
(51) Int. Cl.: A61B 17/068, H02J 7/00

(54) **STAPLER AND TIMED DISCHARGE METHOD THEREOF**

(30) Priority: 13.11.2019 CN 201911104692
(71) Applicant: B. J. Zh. F. Panther Medical Equipment Co., Ltd., Beijing 102209 (CN)
(72) Inventor: LIU, Qing, Beijing 102209 (CN); CHEN, Xiaoqiang, Beijing 102209 (CN); ZHANG, Jianping, Beijing 102209 (CN); LIU, Libo, Beijing 102209 (CN); YANG, Xuelan, Beijing 102209 (CN)
(74) Representative: Huang, Liwei
(86) International application number: PCT/CN2020/101755
(87) International publication number: WO 2021/093356

(57) **Abstract**

Disclosed are an anastomat and a timed discharging method thereof, belonging to the technical field of medical devices. The anastomat includes a body tail plate and a battery pack, a triode and a controller. The elastic sheets for positive and negative signals of the body tail plate are connected to the upper and lower signal conducting sheets in the battery pack by means of a mechanical mechanism, and afterwards are used for outputting a signal for the anastomat. After the power clamping reed of the body tail plate is connected to the power output conducting plate in the battery pack by means of a mechanical structure, the power clamping reed is used for outputting power for the anastomat. The controller is used for controlling the operation of a battery panel in the battery pack, wherein the base of the triode is connected to a pin corresponding to the controller, the collector of the triode is connected to the battery panel, and the emitter of the triode is grounded. The discharging method is implemented based on the anastomat. In the case where it is ensured that electric power can be outputted sufficiently when the battery pack is used, the risk issue of the remaining electric power after the battery is used is solved.

## Description

### Technical Field

The present invention relates to the technical field of medical instruments, and in particular, to an anastomat and a timed discharging method thereof.

### Background Art

An anastomat is the first worldwide stapler in gastrointestinal anastomosis for nearly a century, and had not been widely used for gastrointestinal surgeries until 1978. The anastomat is generally divided into disposable or multi-use anastomat. It is a medically used device for replacing traditionally manual stitching. Due to the development of modern science and technology as well as improvement in the manufacturing technologies, the anastomats used clinically at present are reliable in quality, convenient in use, precise, and tightly suitable, and in particular have advantages such as rapid suturing, simple operation, and quite few side effects and surgical complications. They can also resect tumors that could not be excised in the past in lesions, enjoying great popularity and admiration among Chinese and foreign clinical surgeons. A battery of the battery pack of an electric cavity mirror anastomat in the prior art discharges immediately after being loaded, affecting the battery capacity during use, and reducing the output. After used, the battery pack is not discharging, which easily leads to dangers, thereby raising the requirements for post-processing operation.

### Summary

In view of this, the present invention provides an anastomat and a timed discharging method thereof, which solve the risk issue brought by the remaining electric power after the battery is used, solve the risk issue brought by using a non-original battery, and reduce the requirements for processing after use and the use risk in the case where it is ensured that the electric power can be outputted sufficiently when the battery pack is used, so as to be more suitable for practical use.

In order to achieve the first object above, the technical solution of the assembly device for an anastomat provided by the present invention is as follows:
The anastomat provided by the present invention comprises a body tail plate (313), a battery pack (41), a triode (V3), and a controller.

The elastic sheets (313-2) for positive and negative signals of the body tail plate (313) and the upper and lower signal conducting sheets in the battery pack (41) are connected by means of a mechanical mechanism, and afterwards are used for outputting a signal for the anastomat.

A power clamp reed (313-1) of the body tail plate (313) and a power supply output conducting plate (41-1) in the battery pack (41) are connected by means of a mechanical structure, and afterwards are used for outputting power for the anastomat.

The controller is used for controlling the operation of a battery panel in the battery pack (41),
wherein,
the base of the triode (V3) is connected to a pin corresponding to the controller,
the collector of the triode (V3) is connected to the battery panel, and
the emitter of the triode (V3) is grounded.

The anastomat provided by the present invention can be further implemented by the following technical measures.

Preferably, the anastomat further comprises a protection resistor (R2).

The protection resistor (R2) is connected between the controller and the base of the triode (V3),
wherein,
one end of the protection resistor (R2) is connected to a pin corresponding to the controller, and
the other end of the protection resistor (R2) is connected to the base of the triode (V3).

Preferably, the anastomat further comprises a voltage division resistor,
the voltage dividing resistor being connected between the collector of the triode (V3) and the power supply,
wherein, the voltage dividing resistor is composed of a plurality of resistors connected in parallel.

One end of the voltage dividing resistor is connected to the collector of the triode (V3), and
the other end of the voltage division resistor is connected to the power supply.

Preferably, the model of the controller is STM 32F03.

Preferably, the battery pack (41) and the body tail plate (313) are detachably connected together.

Preferably, a notch (5) is arranged at the tail end of the body tail plate (313), and a protrusion (6) is arranged at the outer wall of the battery pack (41). The notch (5) is adapted to the shape of the protrusion (6).

Preferably, an interface between the notch (5) and the protrusion (6) is in a streamlined shape.

Preferably, the anastomat further comprises a damping block (7) which is fixedly connected to the outside of the protrusion (6).

In order to achieve the second object, the technical solution of the timed discharging method of the anastomat provided by the present invention is as follows:
The timed discharging method of the anastomat provided by the present invention is implemented based on the anastomat provided by the present invention, and the timed discharging method of the anastomat comprises the following steps:
after the body tail plate (313) is conducted with the battery pack (41), the controller obtains the conduction duration t.

After the conduction duration t reaches the set duration t0, the controller controls discharging of the battery in the battery pack (41). The controller does not control the battery in the battery pack (41) to stop discharging until the voltage of the battery reaches a safe value or less.

The timed discharging method of the anastomat provided by the present invention can be further implemented by the following technical measures.

Preferably, the controller controls discharging of the battery in the battery pack (41) after the conduction duration t reaches the set duration t0. During this process, the step of monitoring a battery voltage in the battery pack (41) in real time by the controller is further included.

The anastomat and the timed discharging method thereof provided by the present invention can control discharging of a battery in the battery pack 41 by the controller. After the body tail plate 313 is conducted with the battery pack 41, the controller obtains the conduction duration t. After the conduction duration t reaches the set duration t0, the controller controls discharging of the battery in the battery pack 41. The controller controls the battery in the battery pack 41 not to stop discharging until the voltage of the battery reaches the safety value or less. Due to the application of the triode, before the battery discharges below the safety value, the battery can also continue to discharge until below the safe value, even when the battery is pulled out. Therefore, under the premise of ensuring sufficient electric power to be outputted when the battery pack is used, the risk issue caused by the remaining electric power after the battery is used is solved, the risk issue caused by using a non-original battery is solved, and the requirements for treatment processing after use are reduced, and the use risk is reduced.

### Brief Description of the Drawings

Other various advantages and benefits will become apparent to those of ordinary skilled in the art through reading the following detailed description of preferred embodiments. The drawings are used for the purpose of illustrating preferred embodiments only, not considered as limitation on the present invention. Moreover, the same components are denoted by the same reference signs in the whole drawings.

In the drawings:
Fig. 1 is an exploded view of a body tail plate and a battery pack applied in an anastomat according to an embodiment of the present invention.
Fig. 2 is a structural diagram of a matching relationship between a body tail plate, and a power supply clamping reed and elastic sheets for positive and negative signals applied in an anastomat according to an embodiment of the present invention.
Fig. 3 is a structural diagram of a matching relationship between a battery pack, and a power supply output conducting sheet and upper and lower signal conducting sheets applied in an anastomat according to an embodiment of the present invention;
Fig. 4 is a schematic structural diagram where elastic sheets for positive and negative signals and upper and lower signal conducting sheets applied in an anastomat according to an embodiment of the present invention are mechanically connected to each other.
Fig. 5 is a schematic structural diagram where a power supply output conducting sheet and a power supply clamping reed applied in an anastomat according to an embodiment of the present invention are mechanically connected to each other;
Fig. 6 is a schematic diagram of a circuit applied in an anastomat according to an embodiment of the present invention;
Fig. 7 is a flowchart of steps of a timed discharging method of an anastomat according to an embodiment of the present invention;
wherein,
1-gun tube assembly; 21-release dial button; 22-turn knob; 30-housing identification region;
31-fixed handle; 32-drive trigger; 33-confirmation button; 34-indicator lamp;
35-manual reset cover; 41-battery pack; 41-1-power supply output conducting sheet; 41-2-upper and lower signal conducting sheets; 313-body tail plate; 313-1-power supply clamping reed; 313-2-elastic sheets for positive and negative signals;
5-disposable nail cartridge assembly.

### Detailed Description

In order to solve the problems existing in the prior art, the present invention provided an anastomat and a timed discharging method thereof, which solved the risk issue brought by the remaining electric power after the battery was used, solved the risk issue brought by the use of a non-original battery, and reduced the requirements for processing after use and the use risk in the case where it was ensured that the electric power could be outputted sufficiently when the battery pack was used, so as to be more suitable for practical use.

To further illustrate the technical means adopted by the present invention for achieving the predetermined purpose of the invention and efficacy, the anastomat and the timed discharging method thereof provided according to the present invention would be described below in details by combining the accompanying drawings and preferred embodiments. In the following description, different 'one embodiment's or 'an embodiment's do not necessarily mean the same embodiment. Furthermore, features, structures, or characteristics in one or more embodiments may be combined in any suitable form.

The term "and/or" herein was merely an association relationship describing associated objects, and indicated that there may be three relationships, for example, A and/or B, and it was specifically understood as follows: the relationships may include A and B at the same time, A may exist separately, or B may exist separately, and any one of the foregoing three cases may be provided.

### Embodiment 1

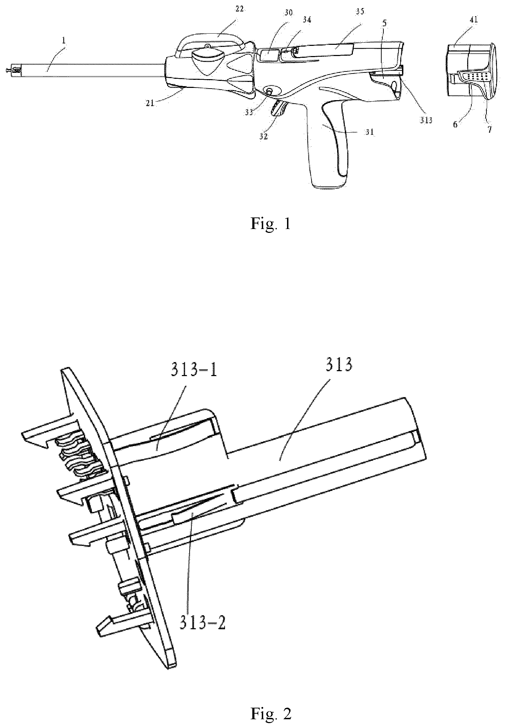

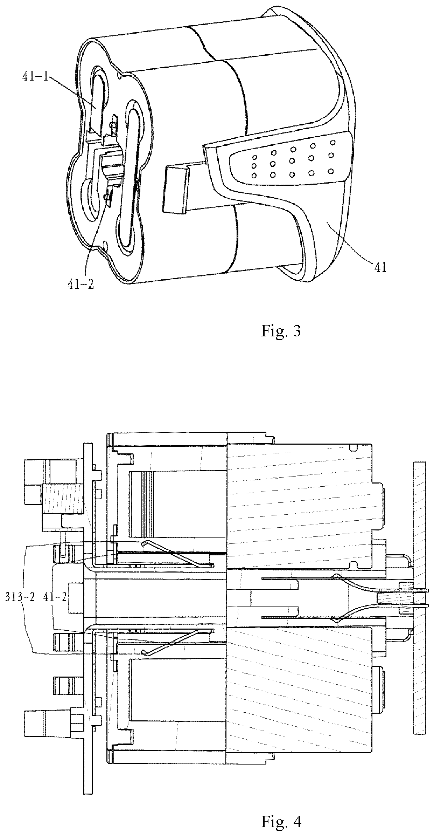

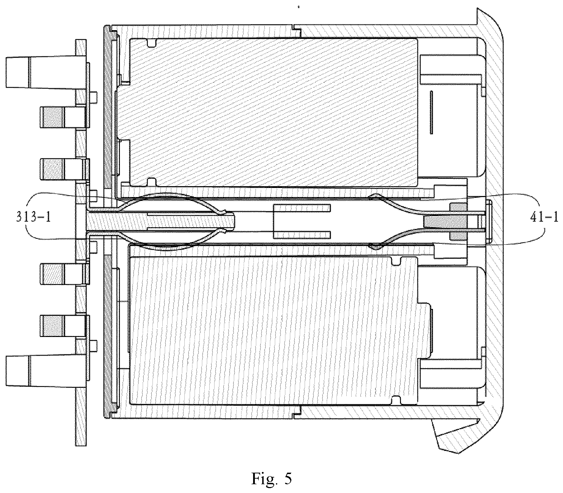

Referring to Figs 1-6, the anastomat provided by Embodiment 1 of the present invention comprised a body tail plate 313 and a battery pack 41, a triode V3 and a controller. The elastic sheets 313-2 for positive and negative signals of the body tail plate 313 were connected to upper and lower signal conducting sheets 42-2 in the battery pack 41 by means of a mechanical mechanism, and afterwards is used for outputting a signal for the anastomat. The power clamping reed 313-1 of the body tail plate 313 was connected to the power supply output conducting sheet 41-1 in the battery pack 41 by means of a mechanical structure, and afterwards was used for outputting power for the anastomat. The controller was used for controlling the operation of a battery panel in the battery pack 41.

The anastomat and the timed discharging method thereof provided by Embodiment 1 of the present invention could control discharging of the battery in the battery pack 41 through the controller. After the body tail plate 313 was conducted with the battery pack 41, the controller obtained the conduction duration t. After the conduction duration t reached the set duration t0, the controller controlled discharging of the battery in the battery pack 41. The controller did not control the battery in the battery pack 41 to stop discharging until the voltage of the battery reached the safety value or less. Due to the application of the triode, before the battery discharged below the safety value, the battery could also continue to discharge until below the safe value, even when the battery was pulled out. Therefore, under the premise of ensuring sufficient electric power to be outputted when the battery pack was used, the risk issue caused by the remaining electric power after the battery was used was solved, the risk issue caused by using a non-original battery was solved, and the requirements for treatment processing after use are reduced, and the use risk is reduced.

The base of the triode V3 was connected to a pin corresponding to the controller, the collector of the triode V3 was connected to the battery panel, and the emitter of the triode V3 was grounded.

The anastomat further included a protection resistor R2. The protection resistor R2 was connected between the controller and the base of the triode V3, wherein one end of the protection resistor R2 was connected to the pin corresponding to the controller, and the other end of the protection resistor R2 was connected to the base of the triode V3. The use of the protection resistor R2 could reduce the current flowing through the triode V3, thereby preventing the triode V3 from being burned due to an excessive current.

The anastomat further included a voltage dividing resistor which was connected between the collector of the triode V3 and the power supply, wherein the voltage dividing resistor was composed of a plurality of resistors connected in parallel with one end of the voltage dividing resistor connected to a collector of the triode V3, and the other end of the voltage dividing resistor connected to a power supply. In this case, the larger the number of parallel resistors of the access circuit was, the smaller the resistance value of the equivalent resistor would be, and the smaller the divided voltage would be. Conversely, the smaller the number of parallel resistors of the access circuit was, the larger the resistance value of the equivalent resistor would be, and the larger the divided voltage would be. In practice, a circuit breaker may also be arranged on the lines of the respective parallel resistors, and the turn-on and turn-off of the respective circuit breakers were controlled by a knob or a plurality of switches, so as to change the resistance value of the equivalent resistor of the voltage dividing resistor access circuit and control the voltage divided by the voltage dividing resistor.

The model of the controller was STM32F03. The STM 32F series belonged to a 32-bit ARM microcontroller at the middle and lower ends. The series of chips were delivered by STMicroelectronics (ST), and the core thereof was Cortex-M3. The series of chips could be divided into three large categories: small capacities (16K and 32K), medium capacities (64K and 128K), large capacities (256K, 384K and 512K) according to the size of the in-chip Flash, and had Chip integration timer, CAN, ADC, SPI, I2C, USB, UART, and various functions.

The battery pack 41 and the body tail plate 313 were detachably connected together, and in this case, the battery pack 41 could be conveniently detached from the body tail plate 313 to facilitate the replacement of a battery in the battery pack 41.

A notch 5 was arranged at the tail end of the body tail plate 313, and a protrusion 6 was arranged at the outer wall of the battery pack 41. The notch 5 was adapted to the shape of the protrusion 6. In this case, it was possible to reduce the risk of destruction due to accidental force when the body tail plate 313 and the battery pack 41 were respectively used.

An interface between the notch 5 and the protrusion 6 was in a streamlined shape, which could not only increase the aesthetic feeling, but could also reduce stress concentration, thereby improving the service lives of the body tail plate 313 and the battery pack 41.

The anastomat further comprised a damping block 7 which was fixedly connected to the outside of the protrusion 6. In this case, when it was necessary to remove the battery pack 41 from the body tail plate 313, the frictional force between the battery pack 41 and a human hand could be increased by grasping the damping block 7, so that the force of detaching the battery pack 41 from the body tail plate 313 was smaller and the operation was easier.

### Embodiment 2

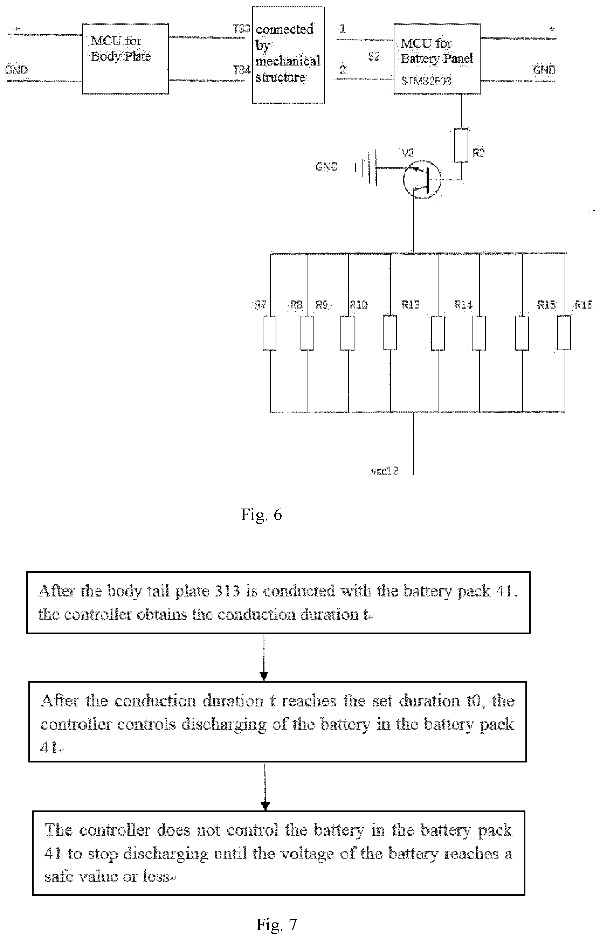

Referring to Fig. 7, the timed discharging method of the anastomat provided by Embodiment 2 of the present invention was implemented based on the anastomat provided by the present invention, the timed discharging method of the anastomat comprising the following steps:
Step S1: After the body tail plate 313 was conducted with the battery pack 41, the controller obtained the conduction duration t.
Step S2: After the conduction duration t reached the set duration t0, the controller controlled discharging of the battery in the battery pack 41.
Step S3: The controller did not control a battery in the battery pack 41 to stop discharging until the voltage of the battery reached a safe value or less.

The timed discharging method of the anastomat provided by Embodiment 2 of the present invention could control discharging of the battery in the battery pack 41 by the controller. After the body tail plate 313 was conducted with the battery pack 41, the controller obtained the conduction duration t. After the conduction duration t reached the set duration t0, the controller controlled discharging of the battery in the battery pack 41. The controller did not control the battery in the battery pack 41 to stop discharging until the voltage of the battery reached the safety value or less. Due to the application of the triode, before the battery was discharged below the safety value, the battery could also continue to discharge below the safe value even if the battery was pulled out. Therefore, in the case where it was ensured that the electric power could be outputted sufficiently when the battery pack was used, the risk issue brought by the remaining electric power after the battery was used was solved, the risk issue brought by using a non-original battery was solved, and the processing requirements after use and the use risk were reduced.

After the conduction duration t reached the set duration t0, the controller controlled discharging of the battery in the battery pack 41. During this process, the step of monitoring the battery voltage in the battery pack 41 in real time by the controller was further included. In this case, the controller could control the cut-off timing of discharging of the battery in time.

Although the preferred embodiments of the present invention have been described, those skilled in the art could make additional changes and modifications to these embodiments once the basic inventive concept was known. Hence, the appended claims were intended to be construed as all changes and modifications that included preferred embodiments and belonged to the scope of the invention.

Obviously, those skilled in the art could make various modifications and variations to the present invention without departing from the spirit and scope of the present invention. Thus, if these modifications and variations to the present invention belonged to the scope of the claims of the present invention and their equivalents, the present invention was also intended to include these modifications and variations.

## Claims

1. An anastomat, **characterized in that** it comprises a body tail plate (313), a battery pack (41), a triode (V3) and a controller;
after elastic sheets (313-2) for positive and negative signals in the body tail plate (313) are connected to upper and lower signal conducting sheets in the battery pack (41) by means of mechanical mechanism, the elastic sheets are used for outputting a signal for the anastomat;
after a power clamp reed (313-1) in the body tail plate (313) is connected to a power supply output conducting plate (41-1) in the battery pack (41) by means of mechanical structure, the power clamp reed is used for outputting power for the anastomat;
the controller is used for controlling the operation of a battery panel in the battery pack (41),
wherein,
the base of the triode (V3) is connected to a pin corresponding to the controller,
the collector of the triode (V3) is connected to the battery panel, and
the emitter of the triode (V3) is grounded.

2. The anastomat according to claim 1, **characterized in** further comprising a protection resistor (R2), the protection resistor (R2) being connected between the controller and the base of the triode (V3),
wherein,
one end of the protection resistor (R2) is connected to a pin corresponding to the controller, and
the other end of the protection resistor (R2) is connected to the base of the triode (V3).

3. The anastomat according to claim 1, **characterized in** further comprising a voltage dividing resistor,
the voltage dividing resistor being connected between the collector of the triode (V3) and the power supply,
wherein the voltage dividing resistor is composed of a plurality of resistors connected in parallel;
one end of the voltage dividing resistor is connected to the collector of the triode (V3), and
the other end of the voltage dividing resistor is connected to the power supply.

4. The anastomat according to claim 1, **characterized in that** the controller model is STM32F03.

5. The anastomat according to claim 1, **characterized in that** the battery pack (41) and the body tail plate (313) are detachably connected together.

6. The anastomat according to claim 5, **characterized in that** a notch (5) is arranged at the tail end of the body tail plate (313), and a protrusion (6) is arranged at the outer wall of the battery pack (41); the notch (5) is adapted to the shape of the protrusion (6).

7. The anastomat according to claim 6, **characterized in that** an interface contacting the notch (5) and the protrusion (6) is in a streamlined shape.

8. The anastomat according to claim 6, **characterized in** further comprising a damping block (7) which is fixedly connected to the outside of the protrusion (6).

9. A timed discharging method for an anastomat, by using the anastomat according to any one of claims 1 to 8, and the timed discharging method for the anastomat comprises the following steps:
after the body tail plate (313) is connected with the battery pack (41), the controller obtains the conduction duration t;
after the conduction duration t reaches the set duration t0, the controller controls discharging of the battery in the battery pack (41), and the controller controls the battery in the battery pack (41) not to stop discharging until the voltage of the battery reaches a safe value or less.

10. The timed discharging method for an anastomat according to claim 9,
when the conduction duration t reaches the set duration t0 and when the controller controls discharging of the battery in the battery pack (41), the controller monitors a battery voltage in the battery pack (41) in real time.
